(19) Europäisches Patentamt
European Patent Office
Office européen des brevets

(11) Veröffentlichungsnummer: **0 161 482**
**B1**

(12) # EUROPÄISCHE PATENTSCHRIFT

(45) Veröffentlichungstag der Patentschrift: **13.09.89**

(21) Anmeldenummer: **85104315.8**

(22) Anmeldetag: **10.04.85**

(51) Int. Cl.⁴: **C 07 C 103/44,** C 07 C 143/14, C 07 C 143/63, C 07 C 91/06, C 07 D 215/18, C 07 D 455/04, C 12 Q 1/28

(54) **Fluorierte Anilinderivate und ihre Verwendung.**

(30) Priorität: **11.04.84 DE 3413693**

(43) Veröffentlichungstag der Anmeldung: **21.11.85 Patentblatt 85/47**

(45) Bekanntmachung des Hinweises auf die Patenterteilung: **13.09.89 Patentblatt 89/37**

(84) Benannte Vertragsstaaten: **AT BE CH DE FR GB IT LI LU NL SE**

(56) Entgegenhaltungen:
**EP-A-0 103 784**
**WO-A-80/01081**
**DE-A-3 037 342**
**GB-A-2 069 498**
**NO-B-132 225**

**J. ORG. CHEM., Band 40, Nr. 1, 1975, Seiten 77-81, Washington, D.C., US; T.L. KRUGER et al.: "Preparation and basicities of substituted N,N-diethyl- and N,N-dimethylaniline oxides"**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Batz, Hans- Georg, Dr. rer. nat., Traubinger- Strasse 63, D-8132 Tutzing (DE)**
Erfinder: **Herrmann, Rupert, Dr. rer. nat., Heinrichstrasse 3, D-8120 Weilheim (DE)**

(74) Vertreter: **Huber, Bernhard, Dipl.- Chem., Patentanwälte H. Weickmann, Dr. K. Fincke F.A. Weickmann, B. Huber Dr. H. Liska, Dr. J. Prechtel Möhlstrasse 22 Postfach 860 820, D-8000 München 86 (DE)**

**EP 0 161 482 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft fluorierte Anilinderivate und ihre Verwendung als Kupplungskomponenten in oxidativen Farbbildungsreaktionen.

Die oxidative Farbkupplung (Emerson-Trinder-Reaktion) von Phenolen oder Anilinen mit geeigneten Kupplungspartnern, wie z. B. mit 4-Aminoantipyrin (4-AAP) oder mit Methylbenzthiazolon-hydrazon (MBTH) kann als Nachweis und zur Bestimmung des für die Farbkupplung verwendeten Oxidationsmittels dienen. Diese Reaktion kann als Grundlage für chemische Diagnostizierverfahren in der enzymatischen Analyse oder auch auf anderen Gebieten, wie z. B. in der Immunologie, herangezogen werden, z. B. zur Bestimmung von Glukose, Harnsäure oder Cholesterin in Körperflüssigkeiten durch Bestimmung des Wasserstoffperoxids, das bei der Oxidation dieser Stoffe mit Enzymen, wie Glukoseoxidase, Urikase und Cholesterinoxidase gebildet wird, zur Substrat- und/oder Peroxidasen-Bestimmung mit Wasserstoffperoxid als Oxidationsmittel oder aber zur Bestimmung von Peroxiden, wie z. B. Lipidperoxiden in Körperflüssigkeiten (vgl. z. B. DE-OS-3 037 342). Eine wesentliche Voraussetzung für derartige Bestimmungsmethoden ist eine hohe Empfindlichkeit, d. h. die Bildung von Farbstoffen mit einem hohen Extinktionskoeffizienten und in hoher Ausbeute. Dies ist z. B. insbesondere in der klinisch-chemischen Diagnostik bei der Bestimmung von Substanzen, die nur in geringen Mengen in Körperflüssigkeiten vorkommen, wichtig, da hierbei Störungen durch z. B. Serumbestandteile, weitgehend ausgeschlossen werden können; ebenso wie in der klinisch-chemischen Diagnostik besteht aber auch auf anderen Gebieten, z. B. in der Immunologie, wo oft Peroxidase als Markerenzym eingesetzt wird, ein Bedarf an empfindlichen chromogenen Systemen.

Es ist bekannt, daß man bei der oxidativen Farbkupplung von Phenolen mit zur Farbbildung geeigneten Kupplungspartnern, wie z. B. mit 4-Aminoantipyrin (4-AAP) oder Methylbenzthiazolonhydrazon (MBTH) die - auf die Oxidationsmittelmenge bezogene - Farbausbeute erhöhen kann, wenn man in 4-Stellung chlorierte oder bromierte Phenole in die Kupplungsreaktion einsetzt. Dieser Effekt beruht darauf, daß es sich bei der Farbbildungsreaktion mit halogenierten Phenolen um einen 2-Elektronen-Oxidationsprozeß handelt, während die in 4-Stellung unsubstituierten Phenole in einem 4-Elektronen-Oxidationsprozeß in die farbigen Verbindungen übergeführt werden (vgl. z. B. die japanische Patentpublikation 9821/79). Die Empfindlichkeit bei der Bestimmung von Oxidationsmitteln, wie z. B. $Fe^{3+}$ oder enzymatisch gebildetem $H_2O_2$, wird also verdoppelt, wenn man 4-Halogenphenole bei der Farbbildungsreaktion verwendet.

Im Gegensatz zu den Halogenphenolen zeigen Anilinderivate, die in 4-Stellung mit Chlor oder Brom substituiert sind, bei der oxidativen Farbkupplung mit 4-AAP oder MBTH keine oder nur eine geringe Farbbildung (vgl. J. Org. Chem. 3 (1938) 153; Analytical Chemistry 33 (1961) 722); während die mit Wasserstoff substituierten aber sonst analogen Verbindungen, in der Regel, insbesondere im neutralen bis schwach sauren pH-Bereich, Farbstoffe mit höheren Extinktionskoeffizienten und längerwelligen Absorptionsmaxima als Phenole liefern. Auch mit N-substituierten Anilinen, die in o- oder p-Stellung durch eine Niederalkylgruppe substituiert sind, werden hinsichtlich Empfindlichkeit und Farbstabilität keine zufriedenstellenden Ergebnisse erzielt (vgl. DE-AS-2 833 612). Die enzymatische Oxidation von 4-Fluoranilin unter Bildung eines roten Benzochinon-di-(p-fluor-anil)-Farbstoffes verläuft ebenfalls sehr unvollständig (vgl. Inorganic Biochemistry, herausgegeben von G.L. Eichhorn, Elsevier, Amsterdam 1973, Band 1 und 2, S. 1000).

Der Einsatz von 4-Fluoranilin und anderen Fluorderivaten von Anilin wurde zwar auch schon zur Bestimmung von Wasserstoffperoxid, z. B. bei der enzymatischen Oxidation organischer Substanzen, herangezogen (vgl. PCT-Anmeldung WO 80/01081; Clinical Chemistry 28 (1982) No. 2, S. 1962); diese Verfahren basieren aber nicht auf einer oxidativen Farbkupplungsreaktion, sondern es werden die bei der durch Peroxid katalysierten Spaltung einer C-F-Bindung freigesetzten Fluorionen elektrometrisch an einer für Fluorionen selektiven Elektrode gemessen.

Aus der EP-A-0 103 784 ist ein Verfahren zur Farbbildung in Gegenwart von Peroxidasen bekannt, bei dem die Geschwindigkeit der Farbbildung durch Zusatz eines Katalysators (Beschleunigers) erhöht wird. Als Beschleuniger werden substituierte Phenole oder 2,6-Difluoranilin verwendet.

Es hat deshalb nicht an Versuchen gefehlt, neue empfindliche chromogene Systeme zur Bestimmung von Oxidationsmitteln, wie z. B. von Wasserstoffperoxid und Peroxidase (POD), zu entwickeln. Vorgeschlagen wurden z. B. Leukofarbstoffe, die in einem 2-Elektronen-Prozeß mit $H_2O_2$/POD zu farbigen Verbindungen oxidiert werden können. Diese Leukofarbstoffe besitzen gegenüber den Zweikomponentensystemen aber oft den Nachteil, daß sie gegenüber Luftsauerstoff instabil oder nur schlechte Substrate der Peroxidase sind. Bei der oxidativen Farbkupplung führten die Entwicklungen zu verbesserten anilinischen Kupplungskomponenten, wobei die Empfindlichkeitssteigerung dabei meist durch Variation der Substituenten am anilinischen Stickstoff oder am C-3-Atom erzielt wurde (vgl. z. B. DE-OS-3 037 342; DE-AS-2 833 612).

Aufgabe der vorliegenden Erfindung ist deshalb die Bereitstellung neuer Anilinderivate, die bei der oxidativen Farbkupplung mit hoher Ausbeute zu Farbstoffen mit hoher Extinktion und Farbstabilität führen, und die deshalb ein empfindliches chromogenes System bei der oxidativen Farbkupplung bilden. Diese Aufgabe wird mit der vorliegenden Erfindung gelöst.

Gegenstand der Erfindung sind fluorierte Anilinderivate der Formel:

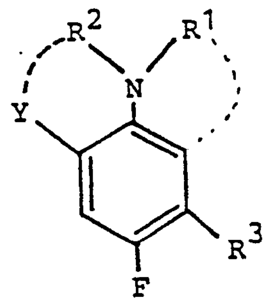

(I)

worin

$R^1$ = H oder $(CH_2)_n$-X, worin n = eine ganze Zahl von 1 bis 3, X = H, OH, $NH_2$, $CH_3CONH$, $CH_3SO_2NH$, $SO_3H$ oder $ArSO_3H$, worin Ar = gegebenenfalls substituierter Arylenrest, $R^1$ alternativ auch eine -$(CH_2)_3$-Gruppe, die an die freie o-Position neben dem N-Atom gebunden ist, Y = H, -O-, -NH-, -S- oder eine C-C-Einfachbindung, $R^2$ für den Fall, daß Y = H, gleich oder verschieden ist von $R^1$ und einen der für $R^1$ angegebenen Reste bedeutet, $R^1$ und $R^2$ aber nicht gleichzeitig H oder $(CH_2)_n$-X, worin X = H ist, bedeuten,

und für den Fall, daß Y eine C-C-Einfachbindung oder -O-, -NH- oder -S- ist, eine mit Y verbundene Alkylengruppe mit 1 bis 3 Kohlenstoffatomen ist, und $R^3$ = H, Alkyl mit 1 bis 3 Kohlenstoffatomen, $OCH_3$, $CH_3CONH$, $CO_2H$ oder $SO_3H$.

Der Rest Ar kann substituiert oder vorzugsweise unsubstituiert sein und ist z. B. 1,4-Naphthylen und insbesondere 1,4-Phenylen; ein substituierter Arylenrest kann einen oder mehrere, vorzugsweise einen oder zwei Substituenten besitzen, wie z. B. Alkyl mit 1 bis 3 Kohlenstoffatomen, OH, $OCH_3$ und/oder Halogen, wie insbesondere Chlor.

$R^2$ in der Bedeutung Alkylen ist Methylen, Ethylen oder Trimethylen, für Y in der Bedeutung C-C-Einfachbindung vorzugsweise Ethylen oder Trimethylen.

Eine Alkylgruppe mit 1 bis 3 Kohlenstoffatomen ist eine Methyl-, Ethyl-, Propyl- oder Isopropylgruppe.

Besonders bevorzugte Verbindungen der Formel I sind das N-(2-Acetamidoethyl)-N-ethyl-4-fluor-3-methylanilin (F-EMAE), N-Ethyl-N-(4-fluor-3-methyl-phenyl)-2-aminoethansulfonsäure (F-EST), N-Ethyl-N-(2-hydroxyethyl)-4-fluor-3-methylanilin (F-EHT), 9-Fluor-julolidin, 9-Fluor-8-methyljulolidin und (6-Fluor-1,2,3,4-tetrahydrochinolin)-N-propansulfonsäure (F-THC-PS).

Es wurde gefunden, daß die fluorierten Anilinderivate der allgemeinen Formel I bei der oxidativen Farbkupplung mit $H_2O_2$/POD und einem geeigneten Kupplungspartner, wie z. B. mit 4-Amino-antipyrin (4-AAP), 4,4'-Diamino-antipyrin (4,4'-DAAP) oder sulfoniertem Methylbenzthiazolonhydrazon (SMBTH), eine gegenüber den nicht fluorierten Analogen um fast das Doppelte erhöhte Empfindlichkeit zeigen; die bei der Kupplung entstehenden Farbstoffe sind dabei die gleichen. Dieses Ergebnis muß als in hohem Maße überraschend angesehen werden, weil es, wie bereits vorstehend besprochen, aus dem Stand der Technik bisher bekannt war, daß in 4-Stellung substituierte Anilinderivate im Gegensatz zu den in 4-Stellung unsubstituierten Analogen bei der oxidativen Farbkupplung keine oder eine geringere Farbbildung zeigen (vgl. auch Fig. 1).

In der nachfolgenden Tabelle 5 werden die bei $\lambda_{max}$ gemessenen Extinktionskoeffizienten ($cm^2/\mu mol\ H_2O_2$) von mit $H_2O_2$/POD oxidierten chromogenen Systemen, die gebräuchliche anilinische Kupplungskomponenten und einen Farbkuppler enthalten, und die Extinktionskoeffizienten sonst gleicher chromogener Systeme, die aber anstelle der gebräuchlichen anilinischen Kupplungskomponenten ein erfindungsgemäßes fluoriertes analoges Anilinderivat enthalten, gegenübergestellt. Als Farbkuppler wurden 4-Amino-antipyrin (4-AAP) 4,4'-Diaminoantipyrin (4,4'-DAAP) und sulfoniertes Methylbenzthiazolonhydrazon (SMBTH) eingesetzt. Der angegebene Quotient $\varepsilon_F/\varepsilon_H$ aus den Extinktionskoeffizienten des oxidierten chromogenen Systems mit fluorierter Kupplungskomponente ($\varepsilon_F$) und des mit der nichtfluorierten Verbindung ($\varepsilon_H$) stellt ein Maß für die Erhöhung der Farbausbeute beim Einsatz der fluorierten Kupplungskomponenten dar.

Die Kupplungsreaktion erfolgt auf an sich bekannte Weise (vgl. z. B. DE-AS-2 833 612); vorzugsweise wird bei Raumtemperatur gearbeitet, wobei sich ein Verhältnis von Kupplungskomponente/Farbkuppler > 5 als besonders zweckmäßig erwiesen hat. Die pH-Werte liegen vorzugsweise im neutralen bis schwach sauren Bereich. Der Tabelle I liegen die folgenden Werte zugrunde (Konzentrationen im Reaktionsgemisch):

Konzentrationen im Reaktionsgemisch:

| | |
|---|---|
| Kalium-Phosphat-Puffer: | 0,1 Mol/l |
| Anilinische Kupplungskomponente: | $3 \times 10^{-1}$ mMol/l |
| Farbkuppler: | $3 \times 10^{-2}$ mMol/l |
| Wasserstoffperoxid: | $1,5 \times 10^{-2}$ mMol/l |

Tabelle I

| Farbkuppler | Kupplungs-komponente | pH-Wert | max | ε ($cm^2/\mu mol\ H_2O_2$) | $\varepsilon_F/\varepsilon_H$ |
|---|---|---|---|---|---|
| 4-AAP | EMAE | | 552 | 16,3 | |
| | F-EMAE | 5,5 | 552 | 29,8 | 1,83 |
| | EST | | 552 | 17,0 | |
| | F-EST | 5,5 | 552 | 30,5 | 1,8 |
| | EHT1) | | 550 | 16,3 | |
| | F-EHT1) | 5,5 | 548 | 29,2 | 1,79 |
| | F-THC-PS2) | 5,5 | 555 | 28,9 | - |
| | Julolidin3) | | 487 | 7,3 | |
| | 9-F-Julolid3) | 5,5 | 546 | 22,8 | - |
| | Julolidin3) | | 544 | 4,7 | |
| | 9-F-Julolid.3) | 7,0 | 547 | 20,3 | 4,3 |
| 4,4'-DAAP | EMAE | | 554 | 17,8 | |
| | F-EMAE | 5,5 | 554 | 32,7 | 1,84 |
| | EST | | 554 | 19,2 | |
| | F-EST | 5,5 | 554 | 33,8 | 1;76 |
| S-MBTH | EMAE | | 570 | 30,4 | |
| | F-EMAE | 5,5 | 570 | 49,8 | 1,64 |
| | EST | | 580 | 30,2 | |
| | F-EST | 5,5 | 580 | 42,0 | 1,4 |
| | 9-F-8-Methyl-julolidin | 5,5 | 525 | 50,6 | - |
| | F-THC-PS | 5,5 | 565 | 51,5 | - |

1) Reaktionsmischung enthält noch 0,15 g/l Triton X-100
2) Die Konzentration der Kupplungskomponente bei diesem Versuch ist 6 x $10^{-2}$ mMol/l
3) Reaktionsmischung enthält noch 1 g/l Triton X-100

Aus der Tabelle I ist ersichtlich, daß mit den erfindungsgemäßen, in 4-Stellung fluorierten Anilinderivaten unter sonst gleichen Bedingungen wesentlich höhere Extinktionswerte (um den Faktor 1,4 bis 1,84) erhalten werden; mit den erfindungsgemäßen Verbindungen werden somit Kupplungskomponenten bereitgestellt, deren Verwendung zusammen mit üblichen Farbkupplern ein sehr empfindliches chromogenes System für die oxidative Farbkupplung ergibt.

Gegenstand der Erfindung ist deshalb auch die Verwendung der erfindungsgemäßen Verbindungen der allgemeinen Formel I,
worin
$R^1$ = H oder $(CH_2)_n$-X,
worin
n = eine ganze Zahl von 1 bis 3,.
X = H, OH, $NH_2$, $CH_3CONH$, $CH_3SO_2NH$, $SO_3H$ oder $ArSO_3H$,
worin
Ar = gegebenenfalls substituierter Arylenrest,
$R^1$ alternativ auch eine $(CH_2)_3$-Gruppe, die an die freie o-Position neben dem N-Atom gebunden ist,
Y = H, -O-, -NH-, -S- oder eine C-C-Einfachbindung,
$R^2$ für den Fall, daß Y = H, gleich oder verschieden ist von $R^1$ und einen der für $R^1$ angegebenen Reste bedeutet, und für den Fall, daß Y eine C-C-Einfachbindung oder -O-, -NH- oder -S- ist, eine mit Y verbundene Alkylengruppe mit 1 bis 3 Kohlenstoffatomen ist, und $R^3$ = H, Alkyl mit 1 bis 3 Kohlenstoffatomen, $OCH_3$, $CH_3CONH$, $CO_2H$ oder $SO_3H$,
als Kupplungskomponenten bei oxidativen Farbbildungsreaktionen, insbesondere zur Bestimmung von

$H_2O_2$ und POD, sowie ein Mittel zur analytischen Bestimmung von oxidierenden Substanzen, insbesondere $H_2O_2$, durch oxidative Farbkupplung, das dadurch gekennzeichnet ist, daß es als Kupplungskomponente eine dieser erfindungsgemäßen Verbindungen der Formel I enthält.

Als Farbkuppler können die für solche Farbkupplungsreaktionen gebräuchlichen Farbkuppler eingesetzt werden; vorzugsweise arbeitet man mit 4-Amino-antipyrin (4-AAP) 4,4,-Diaminoantipyrin (4,4'-DAAP) oder sulfoniertem Methylbenzthiazolonhydrazon (SMBTH).

Die erfindungsgemäßen Verbindungen der Formel I lassen sich nach an sich bekannten Synthesewegen herstellen, z. B. nach einem für die entsprechenden in 4-Stellung unsubstituierten Anilinderivate bekannten Verfahren (vgl. z. B. europäische Patentschrift 0 007 787; DE-AS-2 856 487; DE-OS-3 003 490) unter Verwendung eines entsprechenden fluorierten Ausgangsproduktes.

Fig. 1 zeigt den Vergleich der chromogenen Systeme EMAE/4-Aminoantipyrin und Halogen-EMAE/4-Aminoantipyrin bei der Farbbildung durch Oxidation mit $H_2O_2$/POD. Die Konzentrationen im Reaktionsgemisch entsprechen den oben für Tabelle I angegebenen Werten; zusätzlich enthält das Reaktionsgemisch 0,5 g/l Triton X-100. Die Reaktion wurde durch Zugabe von Peroxidase gestartet. Meßwellenlänge 550 nm. Die Fig. 1 verdeutlicht die überraschenderweise wesentlich höhere Empfindlichkeit bei der Verwendung von erfindungsgemäßen, in 4-Stellung fluorierter Anilinderivate gegenüber den sonst gleichen chromogenen Systemen, die aber ein unsubstituiertes oder durch Chlor oder Brom substituiertes Anilinderivat enthalten.

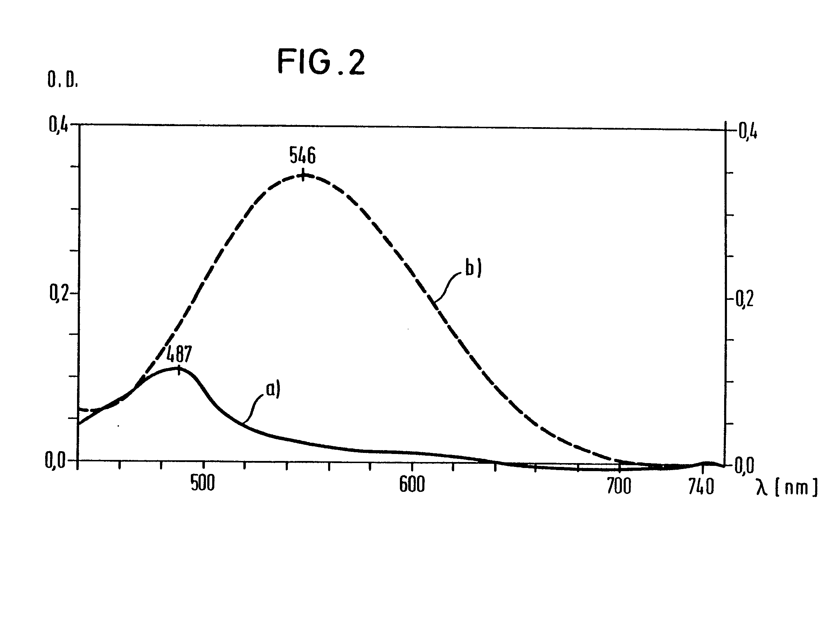

Ein besonderer Vorteil beim Einsatz fluorierter Kupplungskomponenten wird im Falle des Julolidins beobachtet. Wird die oxidative Kupplung von 4-Aminoantipyrin mit Julolidin bei pH 5,5 durchgeführt, so erhält man einen Farbstoff dessen $\lambda_{max}$ nur bei 487 nm liegt, während bei Verwendung von 9-Fluor-julolidin wie üblich ein Farbstoff mit einem $\lambda_{max}$ im Bereich von 550 nm (546 nm) gebildet wird (Fig. 2).

## Beispiele

### Beispiel 1:

N-(2-Acetamidoäthyl)-N-äthyl-4-fluor-3-methylanilin (F-EMAE)

a) N-Äthyl-4-fluor-3-methyl-anilin

Zu einer Suspension von 4,55 g (0,12 Mol) Lithiumaluminiumhydrid in 50 ml Tetrahydrofuran (abs.) wird bei Raumtemperatur während 45 Minuten eine Lösung von 15,05 g (0,09 Mol) N-Acetyl-4-fluor-3-methylanilin in 80 ml THF (abs.) getropft. Nach beendeter Zugabe kocht man 2 Stunden am Rückfluß und dampft anschließend die Reaktionsmischung im Rotationsverdampfer weitgehend ein. Nach Zusatz von 50 ml Äther zerstört man überschüssiges $LiAlH_4$ durch tropfenweise Zugabe von Wasser, trocknet mit Natriumsulfat und saugt ab. Der Filterrückstand wird mit Äther gewaschen, das Filtrat eingedampft und im Kugelrohr (Badtemperatur 155°C) bei 18 Torr destilliert.

Ausbeute: 12,5 g = 91 % d. Th.

$^1$H-NMR ($CDCl_3$): δ = 1,16 (t, J = 7 Hz, 3H); 2,15 (d, J = 2 Hz, 3H); 3,0 (dq, J = 7 Hz; J = 7 Hz, 2H); 5,31 (s br., 1H); 6,39 (ddd, J = 9; 3,5 und 3,5 Hz, 1H, H-6); 6,45 (dd, J = 6,5 Hz, J = 3,5 Hz, 1H, H-2); 6,88 ppm (dd, J = 9 Hz, J = 9 Hz, 1H, H-5).

Elementaranalyse: ber. F 12,40
($C_9H_{12}FN$, 153,2) gef. F 11,97.

b) N-(2-Acetamidoäthyl)-N-äthyl-4-fluor-3-methyl-anilin 7,66 g (50 mMol) N-Äthyl-4-fluor-3-methyl-anilin und 4,68 g (55 mMol) N-Acetylaziridin werden in 100 ml Methanol (abs.) 15 Stunden am Rückfluß gekocht. Anschließend wird die Reaktionsmischung im Rotationsverdampfer eingedampft und der Rückstand mit Diisopropyläther digeriert. Der dabei erhaltene Feststoff wird abgesaugt, mit Diisopropyläther gewaschen und getrocknet.

Ausbeute: 4,65 g = 39 % d. Th.

Fp.: 88°C

$^1$H-NMR (DMSO-$D_6$) : δ = 1,04 (t, J = 7 Hz, 3H); 1,80 (s, 3H); 2,17 (d, J = 2 Hz, 3H); 3,2 (m, 4H); 3,29 (q, J = 7 Hz, 2H); 6,37 - 6,70 (m, 2H); 6,87 (dd, J = 9 Hz; J = 9 Hz, 1H); 7,9 ppm (s br, 1H).

Elementaranalyse: ber. C 65,52 H 8,04 F 7,97 N 11,76 ($C_{13}H_{19}FN_2O$, 238,31) gef. C 65,74 H 8,31 F 8,22 N 11,88

Auf analoge Weise können, ausgehend vom N-Acetyl-4-(chlor- oder brom)-3-methyl-anilin, die Vergleichsverbindungen Cl-EMAE und Br-EMAE erhalten werden.

### Beispiel 2:

N-Äthyl-N-(4-fluor-3-methylphenyl)-2-aminoäthansulfonsäure (F-EST), Na-Salz

Eine Mischung aus 12,25 g (80 mMol) N-Äthyl-4-fluor-3-methylanilin (Beispiel 1a), 18,57 g (88 mMol) 2-Bromäthansulfonsäure-Natriumsalz, 100 mg Kaliumjodid, 29,7 g (160 mMol) Tributylamin und 20 ml DMF wird 12 Stunden bei 170°C Badtemperatur erhitzt. Beim Abkühlen setzt man, bei ca. 90°C, 8 ml 10 N NaOH zu. Nach Zugabe von wenig Aceton fällt im Kühlschrank nicht umgesetztes Bromäthansulfonsäure-Natriumsalz aus und wird durch Filtration abgetrennt. Der nach dem Einengen des Filtrats am Rotationsverdampfer erhaltene Rückstand wird aus Butanol umkristallisiert.

Ausbeute: 5,3 g = 23 % d. Th.

$^1$H-NMR (DMSO-$D_6$) : δ = 1,06 (t, J = 7 Hz, 3H); 2,19 (d, J = 2 Hz, 3H); 2,69 ("t", J = 8 Hz, 2H); 3,30 (q, J = 7 Hz, 2H); 3,52 ("t", J = 8 Hz, 2H); 6,50 (ddd, J = 9; 3,5 und 3,5 Hz, 1H, H-6) 6,57 (dd, J = 6,5 Hz, J = 6,5 Hz, 1H, H-2) 6,93 ppm (dd, J = 9 Hz, J = 9 Hz, 1H, H-5).

Elementaranalyse: ber. C 46,64 H 5,34 F 6,71 N 4,94 S 11,32 Na 8,19 ($C_{11}H_{15}FNNaSO_3$,283,3) gef. C 44,22 H 5,20 F 5,92 N 4,70 S 11,00 Na 8,15.

**Beispiel 3:**

N-Äthyl-N-(4-sulfophenyl-methyl)-4-fluor-3-methyl-anilin, Natrium-Salz

Eine Mischung aus 1,53 g (10 mMol) N-Äthyl-4-fluor-3-methylanilin, 2,73 g (10 mMol) 4-Brommethylbenzolsulfonsäure, Natrium-Salz (hergestellt durch NBS-Bromierung von 4-Toluolsulfonsäurechlorid und anschließende Behandlung mit 1 Äquivalent NaOH) und 2,8 ml Triäthylamin in 90 ml DMF werden 6 Stunden am Rückfluß gekocht. Beim Abkühlen werden 2 ml 10 N Natronlauge zugesetzt, anschließend wird im Vakuum eingedampft. Das aus dem Rückstand durch Umkristallisation aus n-Butanol gewonnene Produkt wird abgesaugt, mit Äther gewaschen und getrocknet.

Ausbeute: 0,1 g

$^1$H-NMR (DMSO-$D_6$) : δ = 1,09 (t, J = 7 Hz, 3H) 2,13 (d, J = 2 Hz, 3H); 3,38 (q, J = 7 Hz, 2H); 4,42 (s, 2H); 6,3 - 6,6 (m, 2H); 6,83 (dd, J = 9 Hz; J = 9 Hz, 1H); 7,15 und 7,53 ppm (AA'BB'-System, J = 9 Hz, 4H).

**Beispiel 4:**

N-Äthyl-N-(2-hydroxyäthyl)-4-fluor-3-methylanilin (F-EHT)

7,66 g (0,05 ) Mol) N-Äthyl-4-fluor-3-methylanilin, 20,1 g (0,25 Mol) 2-Chloräthanol und 15 g (0,15 Mol) Calciumcarbonat in einer Mischung aus 40 ml Dioxan und 20 ml Wasser werden 50 Stunden am Rückfluß gekocht. Nach dem Abkühlen der Reaktionsmischung wird mit Wasser verdünnt und mit Essigester extrahiert. Die organische Phase wird mehrmals mit Wasser gewaschen, mit Natriumsulfat getrocknet und der nach dem Eindampfen erhaltene Rückstand im Kugelrohr (Badtemperatur 145°C) bei 0,1 Torr destilliert.

Ausbeute: 5,3 g = 54 % d. Th.

$^1$H-NMR (DMSO-$D_6$) : δ = 1 ,06 (t, J = 7 Hz, 3H) 2,17 (d, J = 2 Hz, 3H); 3,2 - 3,35 (m, 4H); 3,50 (q, J = 7 Hz, 2H); 4,54 (t, J = 5 Hz, 1H; OH); 6,44 (ddd, J = 9; 3,5 und 3,5 Hz, 1H, H-6); 6,51 (dd, J = 6,5 Hz, J = 3,5 Hz, 1H, H-2); 6,85 ppm (dd, J = 9 Hz, J = 9 Hz, 1H, H-5).

Elementaranalyse: ber. C 66,98 H 8,18 F 9,63 7,10 ($C_{11}H_{16}FNO$, 197,25)gef. C 66,44 H 8,25 F 9,66 N 7,61

**Beispiel 5**

3- (6-Fluor-1 ,2 ,3 ,4-tetrahydro-1-chinolyl)-propansulfonsäure

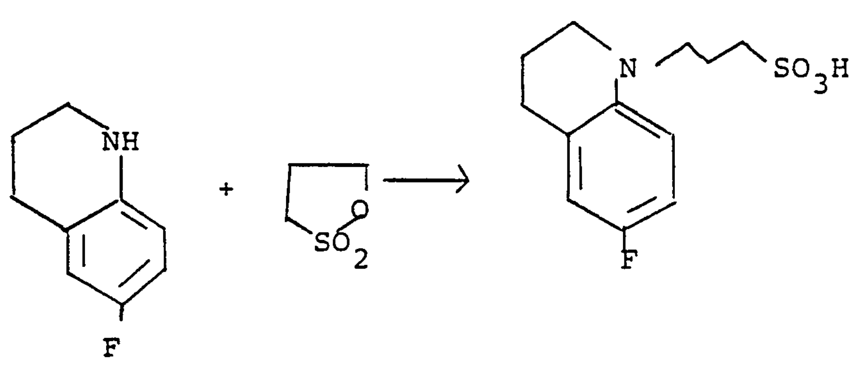

Zu 6,05 g (40 mMol) 6-Fluor-1,2,3,4-tetrahydrochinolin in 20 ml Aceton werden bei 50 bis 60°C 5,9 g (48 mMol) Propansulton, gelöst in 20 ml Aceton, während 40 Minuten zugetropft. Nach 24 Stunden Kochen am Rückfluß und nochmals nach 48 Stunden werden weitere 5,9 g Propansulton zugegeben. Nach insgesamt 60 Stunden refluxieren wird die Reaktionsmischung eingedampft und im Hochvakuum (0,01 Torr) flüchtige Komponenten durch Erhitzen auf ca. 150°C entfernt. Den Rückstand kristallisiert man aus 115 ml Methanol um.

Ausbeute: 4,1 g = 38 % d.Th.

Fp.: 233 bis 236°C

MS (trimethylsilyliertes Derivat): m/e = 345 (M+).

**Beispiel 6**

9-Fluorjulolidin-Hydrochlorid

55,5 g (0,5 mol) 4-Fluoranilin und 472,2 g (3 mol) 1-Brom-3-chlorpropan werden 21 Stunden am Rückfluß gekocht. Beim Abkühlen setzt man bei 50°C 45 ml conc. Salzsäure und 375 ml Wasser zu und entfernt überschüssiges Brom-chlorpropan durch Wasserdampfdestillation. Der Destillationsrückstand wird mit 33-%-iger Natronlauge alkalisch gestellt und zweimal mit Chloroform ausgeschüttelt. Die vereinigten organischen Phasen werden mit Wasser gewaschen, über Natriumsulfat getrocknet und am Rotationsverdampfer eingedampft. Die Reinigung des dabei erhaltenen Rückstands erfolgt durch fraktionierte Destillation (Kp.: 110 - 113C/0,4 Torr).

Ausbeute: 27,9 g = 29 % d. Th.

956 mg (5 mMol/l) 9-Fluorjulolidin in 5 ml Aceton werden unter Eiskühlung mit 497 µl (6 mMol) Salzsäure, 37-%-ig in 5 ml Aceton versetzt. Man saugt das ausgefallene Produkt ab, wäscht mit Aceton und kristallisiert aus Isopropanol um.

Fp.: 221 - 224°C

1H-NMR ($DMSO-D_6$): δ = 2,11 (quintett, J = 7 Hz, 4H); 2,84 (t, J = 7 Hz, 4H); 3,29 ("t" 4H); 5,8 (s.br., 1H); 6,86 ppm (d, J = 9 Hz, 2H).

Elementaranalyse: ber.: C 63,30 H 6,64 N 6,15 $C_{12}H_{15}C1FN$, 227,71) gef.: C 62,89 H 6,62 N 6,09

Figur 2 zeigt die bei oxidativer Kupplung von 4-Aminoantipyrin mit Julolidin (Kurve a) und mit 9-Fluor-julolidin (Kurve b) bei pH 5,5 erhaltenen Extinktionskurven für die gebildeten Farbstoffe. Die Konzentrationen im Reaktionsgemisch entsprechen hierbei den in Tabelle 1 angegebenen Werten. Das Reaktionsgemisch enthält zusätzlich 1 g/l oberflächenaktives Mittel (Triton X100).

**Beispiel 7**

9-Fluor-8-methyljulolidin

11,25 g (0,09 mol) 4-Fluor-3-methylanilin und 85 g (0,54 mol) 1-Brom-3-chlorpropan werden 24 Stunden am Rückfluß gekocht. Nach dem Abkühlen des Reaktionsgemisches auf ca. 50°C gibt man 10 ml conc. Salzsäure und 70 ml Wasser zu und entfernt überschüssiges Brom-chlorpropan durch Wasserdampfdestillation. Der Destillationsrückstand wird mit 33-%-iger Natronlauge alkalisiert und zweimal mit Chloroform ausgeschüttelt. Nach Trocknung der organischen Phase wird eingedampft und ein Teil des Rückstandes säulenchromatographisch über Kieselgel (Elution mit Chloroform→Methanol) gereinigt.

MS: m/e = 205 (M+).

**Patentansprüche** für die Vertragsstaaten: BE, CH; DE, FR, GB, IT, LU, NL, SE

1. Fluorierte Anilinderivate der Formel

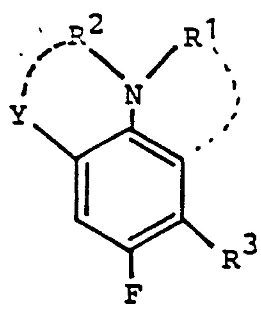

(I)

worin
$R^1$ = H oder $(CH_2)_n$-X, worin n = eine ganze Zahl von 1 bis 3, X = H, OH, $NH_2$, $CH_3CONH$, $CH_3SO_2NH$, $SO_3H$ oder $ArSO_3H$, und Ar = gegebenenfalls substituierter Arylenrest, $R^1$ alternativ auch eine $-(CH_2)_3$-Gruppe, die an die freie o-Position neben dem N-Atom gebunden ist, Y = H, -O-, -NH-, -S- oder eine C-C-Einfachbindung, $R^2$ für den Fall, daß Y = H, gleich oder verschieden ist von $R^1$ und einen der für $R^1$ angegebenen Reste bedeutet, $R^1$ und $R^2$ aber nicht gleichzeitig H oder $(CH_2)_n$-X, worin X = H ist, bedeuten,
und für den Fall, daß Y eine C-C-Einfachbindung oder -O-, -NH- oder -S- ist, eine mit Y verbundene Alkylengruppe mit 1 bis 3 Kohlenstoffatomen ist, und $R^3$ = H, Alkyl mit 1 bis 3 Kohlenstoffatomen, $OCH_3$, $CH_3CONH$, $CO_2H$ oder $SO_3H$.

2. N-(2-Acetamidoethyl)-N-ethyl-4-fluor-3-methyl-anilin.

3. N-Ethyl-N-(4-fluor-3-methyl-phenyl)-2-aminoethansulfonsäure.

4. N-Ethyl-N-(2-hydroxyethyl)-4-fluor-3-methylanilin.

5. (6-Fluor-1,2,3,4-Tetrahydrochinolin)-N-propansulfonsäure.

6. 9-Fluor-julolidin.

7. Verwendung von fluorierten Anilinderivaten der Formel:

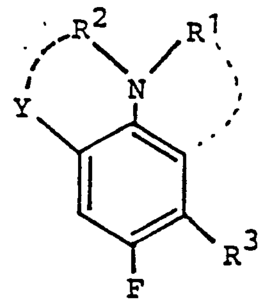

(I)

worin
$R^1$ = H oder $(CH_2)_n$-X, worin n = eine ganze Zahl von 1 bis 3, X = H, OH, $NH_2$, $CH_3CONH$, $CH_3SO_2NH$, $SO_3H$ oder $ArSO_3H$, und Ar 1 = gegebenenfalls substituierter Arylenrest, $R^1$ alternativ auch eine $-(CH_2)_3$-Gruppe, die an die freie o-Position neben dem N-Atom gebunden ist, Y = H, -O-, -NH-, -S- oder eine C-C-Einfachbindung, $R^2$ für den Fall, daß Y = H, gleich oder verschieden ist von $R^1$ und einen der für $R^1$ angegebenen Reste bedeutet, und für den Fall, daß Y eine C-C-Einfachbindung oder -O-, -NH- oder -S- ist, eine mit Y verbundene Alkylengruppe mit 1 bis 3 Kohlenstoffatomen ist, und $R^3$ = H, Alkyl mit 1 bis 3 Kohlenstoffatomen, $OCH_3$, $CH_3CONH$, $CO_2H$ oder $SO_3H$,
als Kupplungskomponente bei oxidativen Farbbildungsreaktionen.

8. Verwendung nach Anspruch 7 zur Bestimmung von Wasserstoffperoxid und POD.

9. Verwendung nach Anspruch 7 oder 8,
dadurch gekennzeichnet,
daß man 4-Aminoantipyrin, 4,4'-Diaminoantipyrin oder sulfoniertes Methylbenzthiazolonhydrazon als Farbkuppler einsetzt.

10. Mittel zur analytischen Bestimmung von oxidierenden Substanzen, insbesondere Wasserstoffperoxid, durch oxidative Farbkupplung, dadurch gekennzeichnet daß es als Kupplungskomponente eine Verbindung der Formel 5 nach Anspruch 7 oder nach einem der Ansprüche 2 bis 6 enthält.

**Patentansprüche** für der Vertragsstaat: AT

1. Verwendung von fluorierten Anilinderivaten der Formel

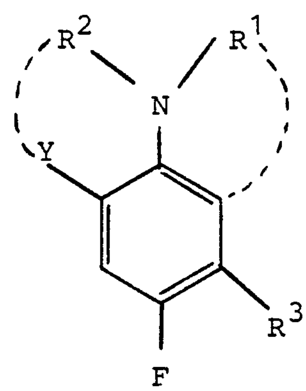

(I)

worin

$R^1$ = H oder $(CH_2)_n$-X, worin n = eine ganze Zahl von 1 bis 3, X = H, OH, $NH_2$, $CH_3CONH$, $CH_3\ SO_2NH$, $SO_3H$ oder $ArSO_3H$, und Ar = gegebenenfalls substituierter Arylenrest, $R^1$ alternativ auch eine $-(CH_2)_3$-Gruppe, die an die freie o-Position neben dem N-Atom gebunden ist, Y = H, -O-, -NH-, -S- oder eine C-C-Einfachbindung, $R^2$ für den Fall, daß Y = H, gleich oder verschieden ist von $R^1$ und einen der für $R^1$ angegebenen Reste bedeutet, und für den Fall, daß Y eine C-C-Einfachbindung oder -O-, -NH- oder -Sist, eine mit Y verbundene Alkylengruppe mit 1 bis 3 Kohlenstoffatomen ist, und $R^3$ = H, Alkyl mit 1 bis 3 Kohlenstoffatomen, $OCH_3$, $CH_3CONH$, $CO_2H$ oder $SO_3H$, als Kupplungskomponente bei oxidativen Farbbildungsreaktionen.

2. Verwendung von N-(2-Acetamidoethyl)-N-ethyl-4-fluor-3-methyl-anilin für den Zweck von Anspruch 1.

3. Verwendung von N-Ethyl-N-(4-fluor-3-methyl-phenyl)-2-aminoethansulfonsäure für den Zweck von Anspruch 1.

4. Verwendung von N-Ethyl-N-(2-hydroxyethyl)-4-fluor-3-methylanilin für den Zweck von Anspruch 1.

5. Verwendung von (6-Fluor-1,2,3,4-Tetrahydrochinolin)-N-propansulfonsäure für den Zweck von Anspruch 1.

6. Verwendung von 9-Fluor-julolidin für den Zweck von Anspruch 1.

7. Verwendung nach Anspruch 1 zur Bestimmung von Wasserstoffperoxid und POD.

8. Verwendung nach Anspruch 1 oder 7,

dadurch gekennzeichnet,

daß man 4-Aminoantipyrin, 4,4'-Diaminoantipyrin oder sulfoniertes Methylbenzthiazolonhydrazon als Farbkuppler einsetzt.

9. Mittel zur analytischen Bestimmung von oxidierenden Substanzen, insbesondere Wasserstoffperoxid durch oxidative Farbkupplung,

dadurch gekennzeichnet,

daß es als erste Kupplungskomponente eine Verbindung der Formel I nach einem der Ansprüche 1 bis 6 und als zweite Kupplungskomponente einen Farbkuppler nach Anspruch 8 enthält.

**Revendications** pour les Etats Contractants: BE, CH, DE, FR, GB, IT, LI, LU, NL, SE

1. Dérivés fluorés de l'aniline répondant à la formule:

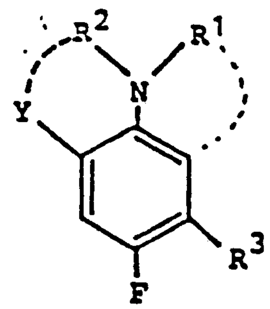

(I)

dans laquelle

$R^1$ = H ou $(CH_2)_n$-X, où n est un nombre entier de 1 à 3, X = H, OH, $NH_2$, $CH_3CONH$, $CH_3SO_2NH$, $SO_3H$ ou $ArSO_3H$, et Ar est un radical arylène substitué le cas échéant, $R^1$ peut aussi être un groupe $-(CH_2)_3$, qui est lié à la position o libre à côté de l'atome de N, Y = H, -O-, -NH-, -S- ou une simple liaison C-C, $R^2$, dans le cas où Y = H, peut être identique à $R^1$ ou être différent de celui-ci et désigne un des radicaux indiqués pour $R^1$, mais $R^1$ et $R^2$ ne sont pas simultanément H ou $(CH_2)_n$-X, où X = H, et dans le cas où Y est une simple liaison C-C ou -O-, -NH- ou -S-, il est un groupe alkylène en $C_1$ à $C_3$ lié à Y, et $R^3$ = H, un groupe alkyle en $C_1$ à $C_3$, $OCH_3$, $CH_3CONH$, $CO_2H$ ou $SO_3H$.

2. N-(2-acétamidoéthyl)-N-éthyl-4-fluoro-3-méthyl-aniline.

3. Acide N-éthyl-N-(4-fluoro-3-méthyl-phényl)-2-aminoéthane-sulfonique.

4. N-éthyl-N-(2-hydroxyéthyl)-4-fluoro-3-méthylaniline.

5. Acide (6-fluoro-1,2,3,4-tétrahydroquinoléine)-N-propanesulfonique.

6. 9-fluoro-julolidine.

7. Utilisation de dérivés fluorés de l'aniline répondant à la formule:

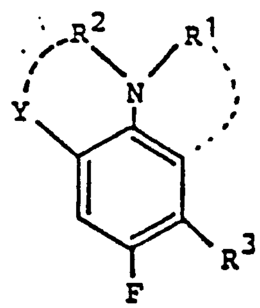

(I)

dans laquelle

$R^1$ = H ou $(CH_2)_n$-X où n est un nombre entier de 1 à 3, X = H, OH, $NH_2$, $CH_3CONH$, $CH_3SO_2NH$, $SO_3H$ ou $ArSO_3H$, et Ar est un radical arylène substitué le cas échéant, $R^1$ peut aussi être un groupe $-(CH_2)_3$ qui est lié à la position o libre à côté de l'atome de N, Y = H, -O-, -NH-, -S- ou une simple liaison C-C, $R^2$, dans le cas où Y = H, est identique à $R^1$ ou est différent de celui-ci et désigne un des radicaux indiqués pour $R^1$, et dans le cas où Y est une simple liaison C-C ou -O-, -NH- ou- -S-, il est un groupe alkylène en $C_1$ à $C_3$ lié à Y, et $R^3$ = H, un groupe alkyle en $C_1$ à $C_3$, $OCH_3$, $CH_3CONH$, $CO_2H$ ou $SO_3H$, comme constituant de copulation dans des réactions de formation de coloration oxydantes.

8. Utilisation selon la revendication 7 pour le dosage du peroxyde d'hydrogène et de la POD.

9. Utilisation selon les revendications 7 ou 8, caractérisée en ce qu'on utilise comme copulant la 4-aminoantipyrine, la 4,4'-diaminoantipyrine ou la méthylbenzthiozolonehydrazone.

10. Agent pour le dosage analytique de substances oxydantes, en particulier du peroxyde d'hydrogène, par population oxydante, caractérisé en ce qu'il contient comme constituant de copulation un composé répondant à la formule I selon la revendication 7 ou selon l'une des revendication 2 à 6.

**Revendications** pour l'Etat Contractant: AT

1. Utilisation de dérivés fluorés de l'aniline répondant à la formule:

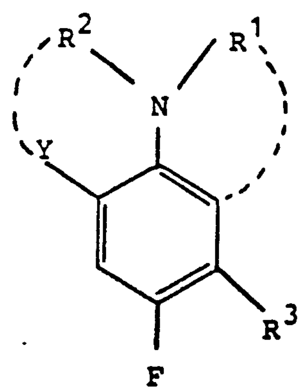

(I)

dans laquelle

$R^1$ = H ou $(CH_2)_n$-X, où n est un nombre entier de 1 à 3, X = H, OH, $NH_2$, $CH_3CONH$, $CH_3SO_2NH$, $SO_3H$ ou $ArSO_3H$, et Ar est un radical arylène substitué le cas échéant, $R^1$ peut aussi être un groupe $-(CH_2)_3$ qui est lié à la position o libre près de l'atome de N, Y = H, -O-, -NH-, -S- ou une simple liaison C-C, $R^2$, dans le cas où Y = H, est identique à $R^1$ ou est différent de celui-ci et désigne un des radicaux indiqués pour $R^1$, et dans le cas où Y est une simple liaison C-C ou -O-, -NH- ou -S-, il est un groupe alkylène en $C_1$ à $C_3$ lié à Y, et $R^3$ = H, un groupe alkyle en $C_1$ à $C_3$, $OCH_3$, $CH_3CONH$, $CO_2H$ ou $SO_3H$ comme constituant de copulation dans des réactions de formation de coloration oxydantes.

2. Utilisation de la N-(2-acétamidoéthyl)-N-éhyl-4-fluoro-3-méthyl-aniline dans l'application de la revendication 1.

3. Utilisation de l'acide N-éthyl-N-(4-fluoro-3-méthyl-phényl)-2-aminoéthane-sulfonique pour l'application de la revendication 1.

4. Utilisation de la N-éthyl-N-(2-hydroxyéthyl)-4-fluoro-3-méthylaniline pour l'application de la revendication 1.

5. Utilisation de l'acide (6-fluoro-1,2,3,4-tétrahydroquinoléine)-N-propanesulfonique pour l'application de la revendication 1.

6. Utilisation de la 9-fluoro-julolidine pour l'application de la revendication 1.

7. Utilisation selon la revendication 1 pour le dosage du peroxyde d'hydrogène et de la POD.

8. Utilisation selon la revendication 1 ou 7, caractérisée en ce qu'on utilise comme copulant la 4-aminoantipyrine, la 4,4'-diaminoantipyrine ou la méthylbenzthiazolonehydrazone.

9. Agent pour le dosage analytique de substances d'oxydants, en particulier du peroxyde d'hydrogène, par copulation oxydante, caractérisé en ce qu'il contient comme premier constituant de copulation un composé répondant à la formule selon l'une des revendications 1 à 6 et comme second constituant de copulation un copulant selon la revendication 8.

**Claims** for the Contracting States: BE, CH, DE, FR, GB, IT, LI, LU, NL, SE

1. Fluorinated aniline derivatives of the formula:

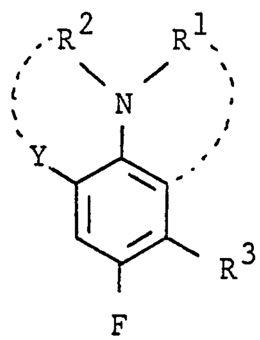

(I)

wherein

$R^1$ = H or $(CH_2)_n$-X, wherein n = a whole number of 1 to 3, X = H, OH, $NH_2$, $CH_3CONH$, $CH_3SO_2NH$, $SO_3H$ or $ArSO_3H$, Ar = optionally substituted arylene radical, $R^1$ is alternatively also a $-(CH_2)_3-$ group, which is bound to the free o-position next to the N-atom, Y = H, -O-, -NH-, -S- or a C-C single bond, $R^2$ in the case that Y = H is the same as or different from $R^1$ and represents one of the radicals given for $R^1$ but $R^1$ and $R^2$ do not simultaneously signify H or $(CH_2)_n$-X wherein X = H and for the case that Y is a C-C single bond or -O-, -NH- or -S-, is an alkylene radical with 1 to 3 carbon atoms bound with Y and $R^3$ = H alkyl with 1 to 3 carbon atoms, $OCH_3$, $CH_3CONH$, $CO_2H$ or $SO_3H$.

2. N-(2-Acetamidoethyl)-N-ethyl-4-fluoro-3-methylaniline.

3. N-Ethyl-N- (4-fluoro-3-methylphenyl -2-aminoethanesulphonic acid.

4. N-Ethyl-N-(2-hydroxyethyl)-4-fluoro-3-methylaniline.

5. (6-Fluoro-1,2,3,4-tetrahydroquinoline)-N-propanesulphonic acid.

6. 9-Fluorojuloidine.

7. Use of fluorinated aniline derivatives of the formula:

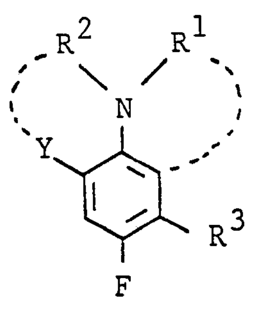

(I)

wherein $R^1$ = H or $(CH_2)_n$-X, wherein n = a whole number from 1 to 3, X = H, OH, $NH_2$, $CH_3CONH$, $CH_3SO_2NH$, $SO_3H$ or $ArSO_3H$ and Ar = optionally substituted arylene radical, $R^1$ is alternatively also a $-(CH_2)_3$ group which is bound to the free o-position next to the N-atom, Y = H, -O-, -NH-, -S- or a C-C single bond, $R^2$ for the case that Y = H is the same as or different from $R^1$ and signifies one of the radicals given for $R^1$ and for the case that Y is a C-C single bond or -O-, -NH- or -S- is an alkylene group with 1 to 3 carbon atoms bound with Y and $R^3$ = H, alkyl with 1 to 3 carbon atoms, $OCH_3$, $CH_3CONH$, $CO_2H$ or $SO_3H$, as coupling components in the case of oxidative colour forming reactions.

8. Use according to claim 7 for the determination of hydrogen peroxide and POD.

9. Use according to claim 7 or 8, characterised in that one uses 4-aminoantipyrine, 4,4'-diaminoantipyrine or sulphonated methylbenzthiazolone hydrazone as colour coupler.

10. Agent for the analytical determination of oxidising substances, especially of hydrogen peroxide, by oxidative coupling, characterised in that as coupling component it contains a compound of formula I according to claim 7 or according to one of claims 2 to 6.

**Claims** for the Contracting State: AT

1. Use of fluorinated aniline derivatives of the formula:

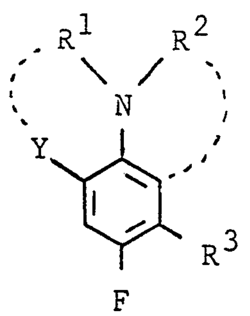

(I)

wherein

$R^1$ = H or $(CH_2)_nX$, wherein n = a whole number from 1 to 3, X = H, OH, $NH_2$, $CH_3CONH$, $CH_3SO_2NH$, $SO_3H$ or $ArSO_3H$ and Ar = optionally substituted arylene radical, $R^1$ alternatively also a $-(CH_2)_3-$ group which is bound to the free o-position next to the N-atom, Y = H, -O-, -NH-, -S- or a C-C single bond, $R^2$ for the case that Y = H is the same as or different from $R^1$ and signifies a radical given for $R^1$ and for the case that Y is a C-C single bond or -O-, -NH- or -S- is an alkylene group with 1 to 3 carbon atoms bound with Y and $R^3$ = H, alkyl with 1 to 3 carbon atoms, $OCH_3$, $CH_3CONH$, $CO_2H$ or $SO_3H$, as coupling components in the case of oxidative colour forming reactions.

2. Use of N-(2-acetamidoethyl)-N-ethyl-4-fluoro-3-methylaniline for the purpose of claim 1.

3. Use of N-ethyl-N-(4-fluoro-3-methylphenyl)-2-aminoethanesulphonic acid for the purpose of claim 1.

4. Use of N-ethyl-N-(2-hydroxyethyl)-4-fluoro-3-methylaniline for the purpose of claim 1.

5. Use of (6-fluoro-1,2,3,4-tetrahydroquinoline)-N-propanesulphonic acid for the purpose of claim 1.

6. Use of 9-fluorojulolidine for the purpose of claim 1.

7. Use according to claim 1 for the determination of hydrogen peroxide and POD.

8. Use according to claim 1 or 7, characterised in that one uses 4-aminoantipyrine, 4,4'-diaminoantipyrine or sulphonate methylbenzthiazolone hydrazone as colour coupler.

9. Agent for the analytical determination of oxidising substances, especially of hydrogen peroxide, by oxidative colour coupling, characterised in that it contains as first coupling component a compound of formula I according to one of claims 1 to 6 and as second coupling component a colour coupler according to claim 8.

FIG. 1

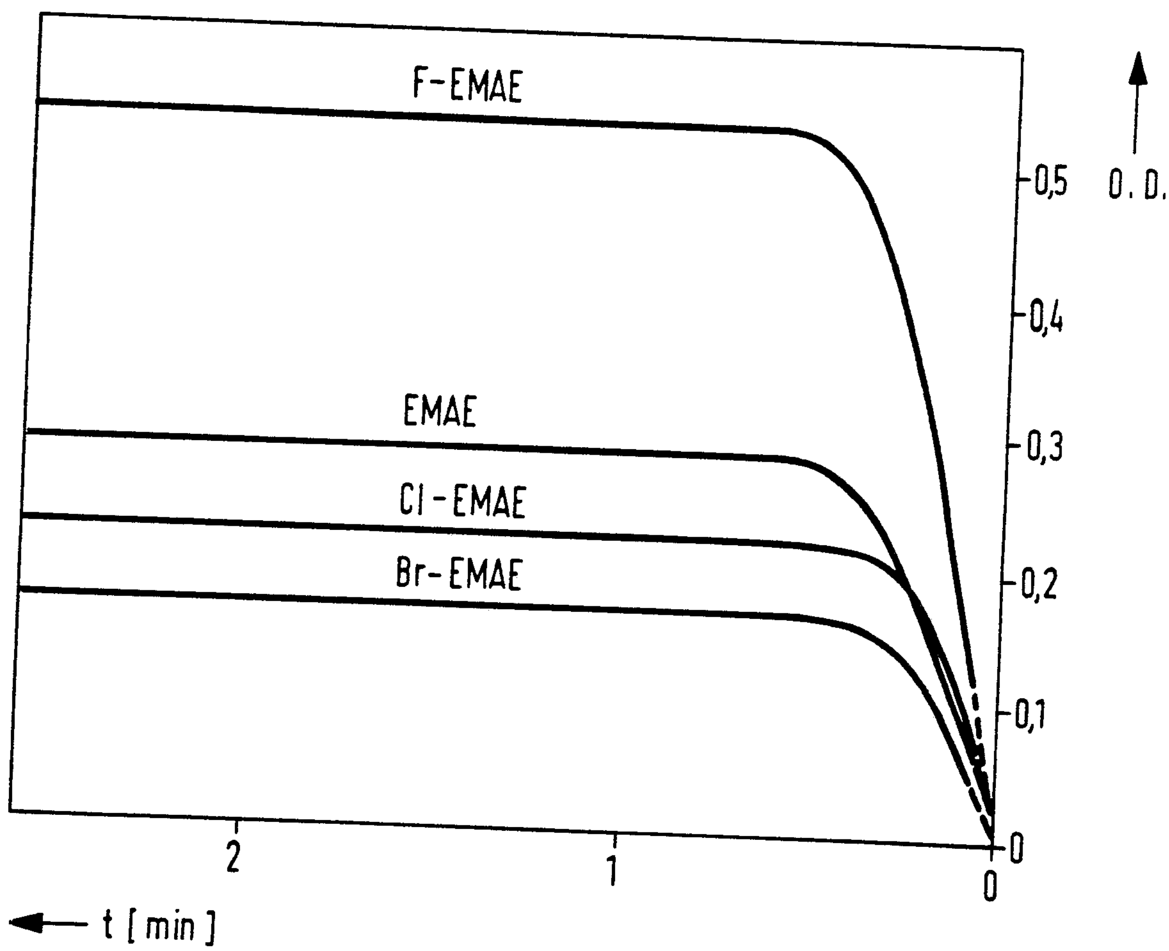

FIG. 2